(19) Europäisches Patentamt
European Patent Office
Office européen des brevets

(11) Veröffentlichungsnummer : **0 051 783**
**B1**

(12) # EUROPÄISCHE PATENTSCHRIFT

(45) Veröffentlichungstag der Patentschrift : **16.11.83**

(51) Int. Cl.[3] : **C 07 C143/665**, C 07 C121/78, B 01 J 23/72

(21) Anmeldenummer : **81108889.7**

(22) Anmeldetag : **24.10.81**

(54) **Verfahren zur Herstellung von 1,4-Diamino-anthrachinon-2-sulfonsäure.**

(30) Priorität : **08.11.80 DE 3042241**

(43) Veröffentlichungstag der Anmeldung : **19.05.82 Patentblatt 82/20**

(45) Bekanntmachung des Hinweises auf die Patenterteilung : **16.11.83 Patentblatt 83/46**

(84) Benannte Vertragsstaaten : **CH DE FR GB LI**

(56) Entgegenhaltungen :
**EP A 0 023 645**
**EP A 0 034 257**
**DE A 2 524 748**
**DE C 1 142 174**
**DE C 1 155 786**

(73) Patentinhaber : **BAYER AG**
**Zentralbereich Patente, Marken und Lizenzen**
**D-5090 Leverkusen 1, Bayerwerk (DE)**

(72) Erfinder : **Kröck, Friedrich Wilhelm, Dr.**
**Ackerstrasse 45**
**D-5068 Odenthal (DE)**
Erfinder : **Neeff, Rütger, Dr.**
**Berta-von-Suttner-Strasse 22**
**D-5090 Leverkusen 1 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 1,4-Diamino-anthrachinon-2-sulfonsaäure

Gegenstand der Erfindung ist ein Verfahren zur Herstellung von 1,4-Diamino-anthrachinon-2-sulfonsäure

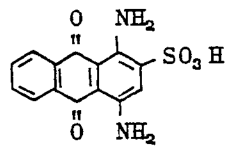

(I)

oder deren Salzen durch Umsetzung von 1-Amino-4-bromanthrachinon-2-sulfonsäure oder deren Salzen mit Ammoniak.

Es ist bereits bekannt, 1,4-Diamino-anthrachinon-2-sulfonsäure oder deren Salze durch Umsetzung von 1-Amino-4-brom-anthrachinon-2-sulfonsäure oder deren Salzen in guter Ausbeute und Reinheit in flüssigem Ammoniak bei 60-100 °C unter einem Druck, bei dem noch flüssiges Ammoniak vorliegt, in Gegenwart von Kupfer oder Kupfersalzen (DE-PS 1 142 174) oder Kupferoxid (DE-PS 1 155 786) herzustellen. Dieses Verfahren weist den Nachteil auf, daß es mit flüssigem Ammoniak ohne weiteres Lösungsmittel in der Nähe der kritischen Temperatur des Ammoniaks durchgeführt wird. Dabei können vor allem im großtechnischen Maßstab durch Produktabscheidungen an den Gefäßwänden und Überhitzung schwerwiegende Komplikationen auftraten.

Es ist weiterhin bekannt, 1-Amino-4-brom-anthrachinon-2-sulfonsäure durch Erhitzen mit Ammoniak bei Gegenwart von Kupfer in die Titelverbindung zu überführen (DE-PS 263 395). Angaben über Reaktionsbedingungen und Ausbeuten fehlen.

Aus der US-PS 3 507 850 (Beispiel 83 in Kombination mit Beispiel 80) ist schließlich bekannt geworden, 1-Amino-4-brom-anthrachinon-2-sulfonsäure mit wäßrigen Aminlösungen bzw. wäßrigem Ammoniak bei 80 °C in Gegenwart von Kupfer (II)-sulfat zu der entsprechenden 1,4-Diaminoverbindung umzusetzen. Auch hier fehlen Angaben über Ausbeute, Reinheit und dgl.

Bei der Nacharbeitung dieser nur unvollkommen beschriebenen Reaktionen im Temperaturbereich von 20-100 °C unter Verwendung von 25 %igem wäßrigem Ammoniak und diversen Kupfersalzkatalysatoren wurde indessen gefunden, daß die Umsetzung nur sehr unvollständig verläuft. Neben einer großen Menge unveränderten Ausgangsmaterials wurden das 4-Hydroxyderivat, das durch « Ullmannkondensation » entstandene 1,1'-Dianthrachinoylderivat und eine Reihe anderer nicht-identifizierter Nebenprodukte erhalten, die sich nur schwer von dem gewünschten 1,4-Diaminoderivat abtrennen lassen.

Diese Beobachtungen stimmen übrigens mit den Feststellungen von anderer Seite im wesentlichen überein (vgl. DE-PS 1 142 174, Spalte 1, oben).

Es wurde nun gefunden, daß man 1,4-Diamino-anthrachinon-2-sulfonsäure bzw. deren Salze, die wichtige Farbstoff-Zwischenprodukte sind, auf einfachem Wege in guter Ausbeute und Reinheit erhält, indem man die Umsetzung der 1-Amino-4-brom-anthrachinon-2-sulfonsäure oder deren Salzen mit Ammoniak in Wasser in Gegenwart von Kupfer, Kupferoxiden und/oder Kupfersalzen bei erhöhter Temperatur und gegebenenfalls in Gegenwart von säurebindenden Mitteln unter einem Ammoniak-Partialdruck von mindestens 5 bar durchführt. Es ist als außerordentlich überraschend zu bezeichnen, daß die Reaktion der 1-Amino-4-brom-anthrachinon-2-sulfonsäure unter den angewandten Reaktionsbedingungen mit Ammoniak in Wasser unter Druck so einheitlich und glatt zu 1,4-Diamino-anthrachinon-2-sulfonsäure verläuft, ohne daß nennenswerte Mengen der 1-Amino-4-hydroxy-anthrachinon-2-sulfonsäure entstehen, wie in DE-PS 1 142 174 berichtet.

1-Amino-4-brom-anthrachinon-2-sulfonsäure und ihre Salze, insbesondere das Natriumsalz sind großtechnische Produkte. Die Menge des Lösungsmittels, Wasser oder wäßriges Ammoniak, kann innerhalb eines größeren Bereichs schwanken und hängt im wesentlichen von der Löslichkeit von Ausgangs- und Endprodukt ab. Auf jeden Fall soll sich ein gut rührbares System bilden, in dem Ausgangs- und Endprodukt vorzugsweise vollständig gelöst sind. Im allgemeinen benötigt man dafür 0,5 bis 5 kg Wasser bzw. wäßriges Ammoniak pro Mol 1-Amino-4-brom-anthrachinon-2-sulfonsäure. Unter wäßrigem Ammoniak wird vorzugsweise die kalt gesättigte Lösung von Ammoniak in Wasser verstanden, d. h. eine ca. 25 bis 30 gewichtsprozentige wäßrige Ammoniaklösung.

Die Reaktion wird beispielsweise bei 40° bis 120 °C, bzw. in einer bevorzugten Ausführungsform bei 60° bis 100 °C, unter einem Ammoniak-Partialdruck von beispielsweise 5 bis 30 bar, vorzugsweise 10 bis 20 bar durchgeführt. Die Ammoniakmenge, die man pro Mol umzusetzender 1-Amino-4-brom-anthrachinon-2-sulfonsäure benötigt, kann zwischen 100 und 2 000 g betragen ; bevorzugt wird eine Menge von 500 bis 1 500 g. Sie wird so bemessen, daß sich der erforderliche Druck bei Reaktionstemperatur einstellt. Unter Praxisbedingungen geht man so vor, daß man die 1-Amino-4-brom-anthrachinon-2-sulfonsäure, bzw. eines ihrer Salze in einem Autoklaven in Wasser oder der wäßrigen Ammoniaklösung zusammen mit dem Kupferkatalysator und gegebenenfalls dem säurebindenden Mittel vorlegt, den Autoklaven verschließt und gegebenenfalls einen so bemessenen Teil des Ammoniaks flüssig oder gasförmig aufpreßt, daß der Druck beim Aufheizen auf Reaktionstemperatur die vorgeschriebene Grenze nicht

überschreitet. Dann wird Ammoniak so nachdosiert, daß der vorgeschriebene Ammoniakdruck während der gesamten Reaktionszeit eingehalten wird.

Geeignete Kupferkatalysatoren sind außer fein verteiltem metallischem Kupfer (sogenannter Kupferbronze) Kupfer(I)-oxid, Kupfer(II)-oxid und Kupfersalze, wie zum Beispiel Kupfercarbonat, basisches Kupfercarbonat, Kupferacetat, basisches Kupferacetat, Kupferformiat, Kupfer(I)-chlorid, Kupfer(II)-chlorid, Kupfer(II)-sulfat, Kupfer(I)-bromid, Kupfer(II)-bromid und andere, sowie Komplexverbindungen des Kupfers und Mischungen von Kupfer oder Kupfersalzen mit Komplexbildnern, wie zum Beispiel Kupfertetramminsulfat, Kupfer/Seignettesalz und andere. Das Kupfer bzw. die Kupferverbindungen kommen in katalytischen Mengen, d. h. in Mengen von 0,1 bis 10 g pro Mol umzusetzender 1-Amino-4-brom-anthrachinon-2-sulfonsäure zum Einsatz.

Als geeignete säurebindende Mittel können gegebenenfalls insbesondere Natriumcarbonat, Natriumhydrogencarbonat, Kaliumcarbonat, Natriumacetat oder Kaliumacetat hinzugefügt werden, wobei man vorzugsweise die der abzuspaltenden HBr-Menge äquivalente Menge des säurebindenden Mittels zusetzt.

Die Reaktionsdauer liegt bei ca. 1 bis 10 Stunden. Danach wird überschüssiges Ammoniak abdestilliert und gegebenenfalls durch Anlegen eines leichten Vakuums weitgehend entfernt.

Das Reaktionsprodukt 1,4-Diamino-anthrachinon-2-, sulfonsaüre kann nach Überprüfung auf Vollständigkeit der Umsetzung zum Beispiel durch Dünnschichtchromatographie direkt in die Lösung weiter umgesetzt werden, beispielsweise mit Cyaniden in grundsätzlich bekannter Weise zu 1,4-Diamino-2,3-dicyano-anthrachinon (vgl. DT-PS 1 108 704, entspr. GB-PS 901 059), oder wird in der üblichen Weise durch Aussäuern oder Aussalzen isoliert.

## Beispiel 1

In einem 0,7 l-Autoklaven legt man die Lösung von 1,2 g Kupfersulfat-pentahydrat in 400 ccm 25-proz. wäßriger Ammoniaklösung vor und trägt unter Rühren 87,4 g 87,5-proz. 1-Amino-4-brom-anthrachinon-2-sulfonsäure (als Natriumsalz ; Gehalt auf freie Säure berechnet) ein. Der Autoklav wird verschlossen, auf 80 °C erhitzt und dann soviel gasförmiges bzw. flüssiges Ammoniak eingepreßt, daß ein Druck von 15 bar erreicht wird. Unter Rühren wird das Gemisch 10 Stunden auf 80 °C erhitzt, wobei man jeweils Ammoniak nachgibt, wenn der Druck um mehr als 0,1 bar absinkt. Insgesamt werden ca. 270 ccm flüssiges Ammoniak (bei Raumtemperatur unter Druck gemessen) eingepreßt. Danach wird der Autoklav abgekühlt, entspannt und überschüssiges Ammoniak durch Evakuieren entfernt. Dann befreit man das Reaktionsgemisch durch Filtration von geringen Mengen ungelöster Anteile. Das Dünnschichtchromatogramm zeigt, daß das Reaktionsgemisch keine 1-Amino-4-bromo-anthrachinon-2-sulfonsäure mehr enthält. Die UV/vis-photometrische Bestimmung ergibt, daß die Lösung 57,4 g 1,4-Diamino-anthrachinon-2-sulfonsäure enthält, das enstpricht einer Ausbeute von 90,2 % d. Th. Die Reaktionslösung wird auf ca. 1 l aufgefüllt und mit konz. Schwefelsäure sauer gestellt, so daß die 1,4-Diamino-anthrachinon-2-sulfonsäure als Sulfat auskristallisiert und der Auslauf der Suspension auf Filtrierpapier nahezu farblos ist. Dann saugt man ab und wäscht das rotbraune Sulfat vorsichtig mit Wasser, bis das zunächst fast farblose Filtrat eine blaßviolette Farbe annimmt. Nach dem Trocknen bei 60 °C erhält man 60,3 g eines Produkts, das laut Analyse zu 92,2 % 1,4-Diamino-anthrachinon-2-sulfonsäure und 0,9 % 1-Amino-4-hydroxy-anthrachinon-2-sulfonsäure besteht und keine 1-Amino-4-bromanthrachinon-2-sulfonsäure mehr enthält. Das enstpricht einer Reinausbeute an isoliertem Produkt von 87,4 %, die mit der UV/vis-photometrisch bestimmten Ausbeute von 90,2 % gut übereinstimmt.

## Beispiel 2

Wiederholt man den Ansatz des Beispiels 1, verschließt den Autoklaven, preßt 180 ccm flüssiges Ammoniak (bei Raumtemperatur unter Druck gemessen) ein, heizt auf 80 °C auf und hält durch Zugabe von weiterem Ammoniak einen Druck von 20 bar aufrecht, wozu man etwa 120 ccm flüssiges Ammoniak benötigt, so erhält man nach Entspannen und Evakuieren entsprechend den Angaben in Beispiel 1 eine Lösung, aus der sich nach Verdünnen mit Wasser durch Fällung mit ca. 100 ccm konz. Schwefelsäure, Absaugen und Waschen wie in Beispiel 1 64,5 g 1,4-Diamino-anthrachinon-2-sulfonsäure als 94,5 %iges Produkt, das keine 1-Amino-4-brom-anthrachinon-2-sulfonsäure und nur 0,2 % 1-Amino-4-hydroxy-anthrachinon-2-sulfonsäure enthält. Das entspricht einer Reinausbeute von 95,8 %.

## Beispiel 3

Wiederholt man den Ansatz des Beispiels 1, verschließt den Autoklaven, preßt 170 ccm flüssiges Ammoniak (bei Raumtemperatur unter Druck gemessen) ein, heizt auf 80 °C auf und hält durch Zugabe von weiterem Ammoniak einen Druck von 15 bar aufrecht, wozu man etwa 120 ccm flüssiges Ammoniak benötigt, so erhält man nach Entspannen und Evakuieren entsprechend den Angaben in Beispiel 1 eine Lösung, die man mit Wasser auf 1 l auffüllt und filtriert. Nach dem Ansäuern des Filtrats mit ca. 100 ccm konz. Schwefelsäure, Absaugen und Waschen wie zuvor erhält man 67,8 g 1,4-Diamino-anthrachinon-2-sulfonsäure als 91,6 %iges Produkt, das keine 1-Amino-4-brom-anthrachinon-2-sulfonsäure und nur 0,3 %

1-Amino-4-hydroxy-anthrachinon-2-sulfonsäure enthält. Das entspricht einer Reinausbeute von 97,6 %.

Beispiel 4

Wiederholt man den Ansatz des Beispiels 1, verschließt den Autoklaven, preßt 100 ccm flüssiges Ammoniak (bei Raumtemperatur unter Druck gemessen) ein, heizt auf 80 °C auf und hält durch Zugabe von weiterem Amoniak einen von 10 bar aufrecht, wozu man etwa 102 ccm flüssiges Ammoniak benötigt, so erhält man nach Entspannen und Evakuieren entsprechend den Angaben in Beispiel 1 eine Lösung, in der sich durch UV/vis-Photometrie ein Gehalt von 58,7 g 1,4-Diamino-anthrachinon-2-sulfonsäure bestimmen läßt. Das entspricht einer Reinausbeute von 92,2 %.

Die erhaltene Lösung wird wie in Beispiel 7 beschrieben für die Herstellung von 1,4-Diamino-2,3-dicyano-anthrachinon verwendet, wobei 55,7 g eines dunkelblauen Pulvers erhalten werden, das laut Analyse 79,4 % 1,4-Diamino-2,3-dicyano-anthrachinon und 10,5 % 1,4-Diamino-2-cyano-anthrachinon enthält, entsprechend einer Reinausbeute an 1,4-Diamino-2,3-dicyano-anthrachinon von 76,7 % bezogen auf 1-Amino-4-brom-anthrachinon-2-sulfonsäure.

Beispiel 5

In einem 3 l-V4A-Autoklaven legt man die Lösung von 6 g Kupfersulfat-pentahydrat in 2 l 25-proz. wäßriger Ammoniaklösung vor und trägt unter Rühren 437 g 87,5-proz. 1-Amino-4-brom-anthrachinon-2-sulfonsäure (als Na-salz; Gehalt auf freie Säure berechnet) ein. Der Autoklav wird verschlossen, 500 ccm gasförmiges oder flüssiges Ammoniak (bei Raumtemperatur unter Druck gemessen) werden eingepreßt und das Reaktionsgemisch wird auf 80 °C unter Rühren erhitzt. Dabei bildet sich ein Druck von 7,8 bar, der durch weitere Zugabe von flüssigem Ammoniak auf 12 bar erhöht wird. Unter Rühren wird das Gemisch 5 Stunden auf 80 °C gehalten, wobei man jeweils Ammoniak nachgibt, wenn der Druck unter 12 bar absinkt. Insgesamt werden noch 282 ccm flüssiges Ammoniak (bei Raumtemperatur unter Druck gemessen) einpreßt. Nach dem Abkühlen, Entspannen und Abziehen des überschüssigen Ammoniaks durch Anlegen eines Vakuums erhält man eine Lösung, die man filtriert und mit Wasser auf 3 250 g auffüllt. Durch UV/vis-Photometrie findet man, daß die Lösung 275,5 g 1,4-Diamino-anthrachinon-2-sulfonsäure enthält ; das enstpricht einer Reaktionsausbeute von 86,6 %. Das Ausgangsprodukt läßt sich in dieser Lösung durch Dünnschichtchromatographie nicht mehr nachweisen. Die erhaltene Lösung wird in Beispiel 10 für die Herstellung von 1,4-Diamino-2,3-dicyano-anthrachinon verwendet. Wird der vorstehende Ansatz wiederholt, und werden zusätzlich in der 25-proz. wäßrigen Ammoniaklösung 53 g wasserfreies Natriumcarbonat gelöst, so erhält man eine Lösung, die laut UV/vis-Photometrie 283,7 g 1,4-Diamino-anthrachinon-2-sulfonsäure, entsprechend einer Reaktionsausbeute von 89,2 %, enthält. 1-Amino-4-brom-anthrachinon-2-sulfonsäure läßt sich durch Dünnschichtchromatographie nicht mehr nachweisen. Ähnliche Ergebnisse werden auch erhalten, wenn man statt Natriumcarbonat 69 g Kaliumcarbonat, 84 g Natriumhydrogencarbonat, 82 g Natriumacetat oder 98 g Kaliumacetat verwendet.

Beispiel 6

Wiederholt man den Ansatz des Beispiels 1 mit dem Unterschied, daß man statt Kupfersulfat 1,0 g Kupferbronze verwendet, verschließt den Autoklaven, heizt unter Rühren auf 80 °C und preßt soviel flüssiges Ammoniak ein, daß während der gesamten Reaktion ein Druck von 12 bar aufrecht erhalten wird, wozu man insgesamt etwa 240 ccm flüssiges Ammoniak (bei Raumtemperatur unter Druck gemessen) benötigt, so erhält man nach einer Reaktionszeit von 5 Stunden und Aufarbeiten wie in Beispiel 1 eine wäßrige Lösung von 1,4-Diamino-anthrachinon-2-sulfonsäure, die laut Dünnschichtchromatogramm kein Ausgangsmaterial mehr enthält. Die erhaltene Lösung wird in Beispiel 11 für die Herstellung von 1,4-Diamino-2,3-dicyano-anthrachinon verwendet.

Ähnlich gute Ergebnisse werden auch erhalten, wenn man statt Kupferbronze 1,2 g Kupfer(I)-chlorid, das man gegebenenfalls zuvor in 12 g gesättigter Natriumchloridlösung mit einigen Tropfen konz. Salzsäure gelöst hat, 1,0 g Kupfer(I)-oxid, 1,2 g Kupfer(II)-oxid, 1,2 g Kupferacetat, 1,2 g basisches Kupferacetat, 1,2 g Kupfercarbonat, 1,2 g basisches Kupfercarbonat oder 1,5 g Kupfer(I)-bromid als Katalysator verwendet.

Beispiel 7

490 g einer Reaktionslösung gemäß Beispiel 1, entsprechend 28 g reiner 1,4-Diamino-anthrachinon-2-sulfonsäure (UV/vis-photometrisch bestimmt), 6,4 g Natriumacetat krist. (+ 3 $H_2O$), 36 g Natriumcyanid, 0,6 g Ammoniumvanadat und 1 040 ccm Wasser werden innerhalb einer Stunde auf 90 °C erhitzt und 4 Stunden lang bei 90 bis 95 °C gerührt. Dabei wird durch die Mischung ein gleichmäßiger Luftstrom von 5,6 l pro Stunde geleitet. Das entstandene 1,4-Diamino-anthrachinon-2,3-dinitril scheidet sich in Form feiner Kristallnadeln aus. Es wird heiß abgesaugt, mit heißem Wasser gewaschen und getrocknet. Die Ausbeute beträgt 24,8 g. Laut Analyse besteht das Produkt aus 80,6 % 1,4-Diamino-2,3-dicyano-anthrachinon und 11,2 % 1,4-Diamino-2-cyano-anthrachinon ; das entspricht einer Reinausbeute von 78,8 % 1,4-Diamino-2,3-dicyano-anthrachinon, bezogen auf die eingesetzte 1,4-Diamino-anthrachinon-2-sulfonsäure.

Beispiel 8

In ein auf 45 °C erwärmtes Gemisch von 1 000 g einer Reaktionslösung gemäß Beispiel 1, entsprechend 58,5 g reiner 1,4-Diamino-anthrachinon-2-sulfonsäure (UV/vis-photometrisch bestimmt), 13 g Natriumcarbonat, 78 g Natriumcyanid und 2 300 ccm Wasser läßt man eine Lösung von 45,5 g m-nitrobenzolsulfonsaurem Natrium in 481 g Wasser in folgender Weise langsam einfließen : Innerhalb der ersten Stunde trägt man 188,5 g, innerhalb der zweiten Stunde 182 g, innerhalb der dritten Stunde 97,5 g und innerhalb der vierten Stunde 58,5 g der Lösung in das Gemisch ein und rührt sodann noch 30 Minuten nach. Dabei wird das Umsetzungsgemisch innerhalb von 30 Minuten 90 °C erhitzt und 4 Stunden bei dieser Temperatur gehalten. Nun saugt man das Umsetzungsgut heiß ab, wäscht es mit heißem Wasser säurefrei und trocknet es. Man erhält 46,3 g blauschwarze Kristalle, die laut Analyse 78,8 % 1,4-Diamino-2,3-dicyano-anthrachinon und 14,5 % 1,4-Diamino-2-cyano-anthrachinon enthalten. Das entspricht einer Reinausbeute von 68,8 % 1,4-Diamino-2,3-dicyano-anthrachinon, bezogen auf die eingesetzte 1,4-Diamino-anthrachinon-2-sulfonsäure.

Beispiel 9

Eine Lösung von 37 g 94,5-proz. 1,4-Diamino-anthrachinon-2-sulfonsäure aus Beispiel 2, 8 g krist. Natriumacetat, 45 g Natriumcyanid und 0,75 g Natriumvanadat in 1 900 ccm Wasser wir dunter Durchleiten eines gleichmäßigen Luftstromes von ca. 7 l/Stde. in 1 Stunde auf 90 °C erhitzt und 4 Stunden, immer unter Rühren und Belüften bei dieser Temperatur belassen. Dann saugt man heiß ab, wäscht mit heißem Wasser und trocknet. Man erhält 31,1 g eines dunkelblauen Pulvers, das laut Analyse 80,6 % 1,4-Diamino-2,3-dicyano-anthrachinon und 11,2 % 1,4-Diamino-2-cyano-anthrachinon enthält, entsprechend einer Reinausbeute von 79,1 %, bezogen auf eingesetzte 1,4-Diamino-anthrachinon-2-sulfonsäure.

Beispiel 10

650 g der Reaktionslösung aus Beispiel 5, entsprechend 55,1 g 1,4-Diamino-anthrachinon-2-sulfonsäure (UV/vis-photometrisch bestimmt ; das entspricht 76,4 g eingesetzter 100-proz. 1-Amino-4-brom-anthrachinon-2-sulfonsäure), 71 g Natriumcyanid, 1,2 g Natriumvanadat und 2 950 ccm Wasser werden 11 Stunden unter Rühren auf 90 °C erhitzt, wobei man einen gleichmäßigen Luftstrom von ca. 11 l/Stde. durch das Reaktionsgemisch leitet. Dann saugt man heiß ab, wäscht mit heißem Wasser und trocknet. Ausbeute : 54,6 g dunkelblaues Pulver, das nach der Analyse aus 73,4 % 1,4-Diamino-2,3-dicyano-anthrachinon und 8,8 % 1,4-Diamino-2-cyano-anthrachinon besteht. Die auf eingesetzte 1-Amino-4-brom-anthrachinon-2-sulfonsäure bezogene Reinausbeute beträgt also 69,5 % ; auf 1,4-Diamino-anthrachinon-2-sulfonsäure bezogen sind das 80,3 % Reinausbeute.

Beispiel 11

Wiederholt man den vorstehenden Ansatz (Beispiel 10) unter Verwendung der gesamten, mit 2 l Wasser verdünnten Reaktionslösung von Beispiel 6, so erhält man 53,5 g eines dunkelblauen Pulvers, das laut Analyse aus 76,2 % 1,4-Diamino-2,3-dicyano-anthrachinon und 11,1 % 1,4-Diamino-2-cyano-anthrachinon besteht. Die auf eingesetzte 1-Amino-4-brom-anthrachinon-2-sulfonsäure bezogene Reinausbeute beträgt also 70,7 %.

**Ansprüche**

1. Verfahren zur Herstellung von 1,4-Diamino-anthrachinon-2-sulfonsäure und deren Salzen durch Umsetzen von 1-Amino-4-brom-anthrachinon-2-sulfonsäure oder deren Salzen mit wäßriger Ammoniaklösung bei erhöhter Temperatur in Gegenwart von Kupferkatalysatoren und gegebenenfalls in Gegenwart von säurebindenden Mitteln, dadurch gekennzeichnet, daß man die Umsetzung bei einem Ammoniak-Partialdruck von mindestens 5 bar durchführt.

2. Verfahren nach Anspruch 1, dadurch gekennzeichnet, daß man die Umsetzung bei einem Ammoniak-Partialdruck von mindestens 10 bar durchführt.

3. Verfahren nach Anspruch 1, dadurch gekennzeichnet, daß man die Umsetzung bei 40° bis 120 °C durchführt.

4. Verfahren nach Anspruch 1, dadurch gekennzeichnet, daß man die Umsetzung bei 60° bis 100 °C durchführt.

5. Verfahren nach Anspruch 1, dadurch gekennzeichnet, daß man als Kupferkatalysatoren Kupfer, Kupferoxide, Kupfersalze, Komplexverbindungen des Kupfers und/oder Mischungen von Kupfer oder Kupfersalzen mit Komplexbildnern verwendet.

6. Verfahren nach Anspruch 1, dadurch gekennzeichnet, daß man als Kupferkatalysatoren fein verteiltes Kupfer (Kupferbronze), Kupfer(I)-oxid, Kupfer(II)-oxid, Kupfer(II)-sulfat, Kupfer(I)-chlorid, Kupfer(II)-chlorid, Kupfer(II)-acetat, basisches Kupferacetat, Kupfercarbonat, basisches Kupfercarbonat, Kupfer(II)-bromid, Kupfer(I)-bromid oder Kupfertetramminsulfat verwendet.

7. Verfahren nach Anspruch 1, dadurch gekennzeichnet, daß man als Kupferkatalysatoren fein verteiltes Kupfer (Kupferbronze), Kupfer(I)-oxid, Kupfer(II)-oxid, Kupfer(II)-sulfat, Kupfer(I)-chlorid oder Kupferacetat verwendet.

8. Verfahren nach Anspruch 1, dadurch gekennzeichnet, daß man als Kupferkatalysator Kupver(II)-sulfat verwendet.

9. Verwendung der nach dem Verfahren gemäß Anspruch 1 hergestellten 1,4-Diamino-anthrachinon-2-sulfonsäure oder deren Salzen zur Herstellung von 1,4-Diamino-2,3-dicyano-anthrachinon.

10. Verwendung nach Anspruch 9, dadurch gekennzeichnet, daß die 1,4-Diamino-anthrachinon-2-sulfonsäure ohne Zwischenisolierung weiter umgesetzt wird.

**Claims**

1. Process for the preparation of 1,4-diamino-anthraquinone-2-sulphonic acid and its salts by reacting 1-amino-4-bromo-anthraquinone-2-sulphonic acid, or its salts, with aqueous ammonia solution at an elevated temperature in the presence of copper catalysts and, if appropriate, in the presence of acid-binding agents, characterised in that the reaction is carried out under a partial pressure of ammonia of at least 5 bar.

2. Process according to Claim 1, characterised in that the reaction is carried out under an ammonia partial pressure of at least 10 bar.

3. Process according to Claim 1, characterised in that the reaction is carried out at 40° to 120 °C.

4. Process according to Claim 1, characterised in that the reaction is carried out at 60° to 100 °C.

5. Process according to Claim 1, characterised in that the copper catalysts used are copper, copper oxides, copper salts, complex compounds of copper and/or mixtures of copper or copper salts with complex-forming agents.

6. Process according to Claim 1, characterised in that the copper catalysts used are finely divided copper (copper bronze), copper(I) oxide, copper(II) oxide, copper(II) sulphate, copper(I) chloride, copper(II) chloride, copper(II) acetate, basic copper acetate, copper carbonate, basic copper carbonate, copper(II) bromide, copper(I) bromide or copper tetrammine sulphate.

7. Process according to Claim 1, characterised in that the copper catalysts used are finely divided copper (copper bronze), copper(I) oxide, copper(II) oxide, copper(II) sulphate, copper(I) chloride or copper acetate.

8. Process according to Claim 1, characterised in that the copper catalyst used is copper(II) sulphate.

9. Use of the 1,4-diamino-anthraquinone-2-sulphonic acid prepared by the process according to Claim 1, or of its salts, for the preparation of 1,4-diamino-2,3-dicyano-anthraquinone.

10. Use according to Claim 9, characterised in that the 1,4-diamino-anthraquinone-2-sulphonic acid is subjected to the further reaction without intermediate isolation.

**Revendications**

1. Procédé de préparation de l'acide 1,4-diamino-anthraquinone-2-sulfonique et de ses sels par réaction de l'acide 1-amino-4-bromo-anthraquinone-2-sulfonique ou de ses sels avec une solution aqueuse d'ammoniac à chaud en présence de catalyseurs au cuivre et, le cas échéant, en présence d'agents fixant les acides, caractérisé en ce que l'on effectue la réaction sous une pression partielle d'ammoniac d'au moins 5 bars.

2. Procédé selon la revendication 1, caractérisé en ce que l'on effectue la réaction sous une pression partielle d'ammoniac d'au moins 10 bars.

3. Procédé selon la revendication 1, caractérisé en ce que l'on effectue la réaction à une température de 40 à 120 °C.

4. Procédé selon la revendication 1, caractérisé en ce que l'on effectue la réaction à une température de 60 à 100 °C.

5. Procédé selon la revendication 1, caractérisé en ce que l'on utilise en tant que catalyseurs au cuivre le cuivre, des oxydes de cuivre, des sels du cuivre, des composés complexes du cuivre et/ou des mélanges de cuivre ou de sels de cuivre avec des agents complexants.

6. Procédé selon la revendication 1, caractérisé en ce que l'on utilise en tant que catalyseurs au cuivre du cuivre à l'état de fine division (bronze de cuivre), de l'oxyde de cuivre-I, de l'oxyde de cuivre-II, du sulfate de cuivre-II, du chlorure de cuivre-I, du chlorure de cuivre-II, de l'acétate de cuivre-II, de l'acétate de cuivre basique, du carbonate de cuivre, du carbonate de cuivre basique, du bromure de cuivre-II, du bromure de cuivre-I ou le sulfate de tétramine-cuivre.

7. Procédé selon la revendication 1, caractérisé en ce que l'on utilise en tant que catalyseurs au

cuivre à l'état de fine division (bronze de cuivre), de l'oxyde de cuivre-I, de l'oxyde de cuivre-II, du sulfate de cuivre-II, du chlorure de cuivre-I ou de l'acétate de cuivre.

8. Procédé selon la revendication 1, caractérisé en ce que l'on utilise en tant que catalyseur au cuivre le sulfate de cuivre-II.

9. Utilisation de l'acide 1,4-diamino-anthraquinone-2-sulfonique ou ses sels préparés par le procédé selon la revendication 1 pour la préparation de la 1,4-diamino-2,3-dicyano-anthraquinone.

10. Utilisation selon la revendication 9, caractérisé en ce que l'on soumet l'acide 1,4-diamino-anthraquinone-2-sulfonique à la conversion sans l'isoler au préalable.